# EUROPÄISCHE PATENTSCHRIFT

(11) **EP 1 910 252 B1**
(45) Veröffentlichungstag und Bekanntmachung des Hinweises auf die Patenterteilung: **22.08.2012**
(21) Anmeldenummer: 06762420.5
(22) Anmeldetag: 05.07.2006
(51) Int. Cl.: C07C 1/24, C07C 41/09

(54) **DEHYDRATISIERUNG VON ALKOHOLEN ZU ALKENEN ODER ETHERN**
DEHYDRATION OF ALCOHOLS TO FORM ALKENES OR ETHERS
DESHYDRATATION D'ALCOOLS POUR L'OBTENTION D'ALCENES OU D'ETHERS

(30) Priorität: 03.08.2005 DE 102005036457
(43) Veröffentlichungstag der Anmeldung: 16.04.2008
(73) Patentinhaber: Merck Patent GmbH, 64293 Darmstadt (DE)
(72) Erfinder: IGNATYEV, Nikolai, 47058 Duisburg (DE); WELZ-BIERMANN, Urs, 64646 Heppenheim (DE); KOPPE, Karsten, 45768 Marl (DE); BARTHEN, Peter, 47495 Rheinberg (DE); FROHN, Hermann, Josef, 47906 Kempen (DE)
(86) Internationale Anmeldenummer: PCT/EP2006/006554
(87) Internationale Veröffentlichungsnummer: WO 2007/014613

(56) Entgegenhaltungen:
- FR-A1- 2 829 132
- GB-A- 2 387 127
- US-A1- 2003 060 359
- FENG SHI ET AL: "The first non-acid catalytic synthesis of tert-butyl ether from tert-butyl alcohol using ionic liquid as dehydrator" CHEMICAL COMMUNICATIONS., 2003, Seiten 1054-1055, XP002414998 GBCHEMICAL SOCIETY, LONDON.
- SASAKI K ET AL: "A novel greener glycosidation using an acid-ionic liquid containing a protic acid" TETRAHEDRON LETTERS, ELSEVIER, AMSTERDAM, NL, Bd. 44, Nr. 30, 21. Juli 2003 (2003-07-21), Seiten 5605-5608, XP004433807 ISSN: 0040-4039

## Beschreibung

Die vorliegende Erfindung betrifft ein Verfahren zur Dehydratisierung von Alkoholen, Polyalkoholen oder Alkoholaten mit mindestens einer CH-Gruppe in α-Stellung zur Alkoholat- bzw. Alkohol-Funktion zu Alkenen , wobei die Dehydratisierung in lonischen Flüssigkeiten der allgemeinen Formel K⁺A⁻ erfolgt nach Anspruch 1.

Die Herstellung von Alkenen durch Wasserabspaltung aus Alkoholaten, insbesondere Lithium- oder Grignardalkoholaten, ist prinzipiell bekannt und dient beispielsweise zur Synthese von arylsubstituierten Alkenen, die z.B als mesogene Substanzen, Pharmawirkstoffe, Pflanzenschutzmittel, Polymere oder Vorstufen in der Feinchemie oder zur Herstellung entsprechender Ausgangsverbindungen verwendet werden können. Zur Wasserabspaltung wird das Alkoholat, das in α-Position zur Alkoholat-Funktion eine CH-Gruppe aufweist, üblicherweise mit einer Säure umgesetzt. Anschließend muss das verwendete Lösungsmittel destillativ entfernt werden oder zusammen mit dem entstandenen Wasser als Azeotrop abdestilliert werden. Alternativ kann die Dehydratisierung auch heterogen mit Aluminiumoxid als Katalysator bei Temperaturen von 300 bis 400°C durchgeführt werden.

Die oben genannten Verfahren haben den Nachteil, dass entweder hohe Temperaturen aufgewendet werden müssen, die zur Zersetzung der organischen Verbindungen führen können, oder dass große Mengen an organischen Lösungsmitteln eingesetzt werden müssen, die nach Durchführung der Wasserabspaltung aufwendig wieder entfernt und fachgerecht entsorgt werden müssen. Gerade für großtechnische Synthesen erweisen sich damit die beiden genannten Methoden als nachteilig, da insbesondere bei Einsatz von Lösungsmitteln zusätzliche sicherheitstechnische Aspekte zur Vermeidung von Belastungen der Umwelt und zum Brandschutz berücksichtigt werden müssen.

Zur Umgehung des Einsatzes in Lösungsmitteln wird in WO 00/51957 vorgeschlagen, Alkene durch heterogen säurekatalysierte Reaktion von Alkoholen in superkritischen Lösungsmitteln, wie z.B. superkritischem CO₂, Propan, Halogenkohlenwasserstoffen oder Stickstoff, zu gewinnen. Die genannte Verwendung superkritischer Lösungsmittel erweist sich aber als sehr aufwendig, da die Synthese in entsprechenden druckfesten Behältern ertolgen muss und etliche der genannten Gase entweder giftig oder ebenfalls brennbar sind. Aus sicherheitstechnischen und ökonomische Gesichtspunkten ist die Verwendung superkritischer Lösungsmittel damit nicht für Synthesen in kommerziellen Maßstäben gut geeignet.

Feng Shi et al., Chem. Commun. 2003, 1054-1055 beschreibt ein Verfahren zur Synthese von Methyl-tert-butylether, Ethyl-tert-butylether und lsopropyl-tert-butylether aus tert-Butylalkohol und Methanol, Ethanol und Isopropanol, wobei als Dehydrator eine ionische Flüssigkeit verwendet wird.

GB 2387127 A beschreibt eine Mischung aus 3-Butyl-1-methylimidazolium bis(trifluormethylsulfonyl)imid und der korrespondierenden Säure H[N(SO₂CF₃)₂].

US 2003/060359 A1 beschreibt Mischungen einer Bronsted-Säure HB und einer lonischen Flüssigkeit Q⁺A⁻, wobei Q⁺ ein organisches Kation bedeutet und A⁻ ein Anion bedeutet, welches nicht identisch zum Anion B oder identisch zum Anion B sein kann als katalytisches System.

FR 2829132 A1 beschreibt eine Mischung aus einer Brönsted-Säure und einer lonischen Flüssigkeit, insbesondere 3-Butyl-1-methylimidazolium bis(trifluormethylsulfonyl)imid und die korrespondierende Säure H[N(SO₂CF₃)₂], zur Oligomerisierung von Olefinen.

Kaname Sasaki et al, Tetrahedron Letters 2003, 44, 5605-5608 beschreibt eine Mischung aus einer Säure und einer Ionischen Flüssigkeit, insbesondere 1-n-Hexyl-3-methylimidazolium bis(trifluormethylsulfonyl)imid und die korrespondierenden Säure H[N(SO₂CF₃)₂], zur Verwendung in einer Glycosylierungsreaktion von Glucopyranosyldiethylphosphit und einem Alkohol.

WO 2007/012825 ist ein Dokument gemäß Artikel 54(3) EPÜ und beschreibt Dehydratisierungsreaktionen von Alkoholen, speziell von Butanol, Ethanol, Methanol, Isopropanol und Pentanol.

Aufgabe der vorliegenden Erfindung war es daher, ein Verfahren der eingangs genannten Art bereitzustellen, das die Synthese der gewünschten Alkene oder von Ethern in sehr hohen Ausbeuten ohne Verwendung flüchtiger Lösungsmittel ermöglicht, das gut beherrschbar ist, keinen hohen sicherheitstechnischen Aufwand erfordert und damit auch eine ökonomische Synthese von Alkenen in kommerziellen Maßstäben erlaubt.

Die oben genannte Aufgabe wird überraschenderweise durch ein Verfahren gemäß der vorliegenden Erfindung gelöst. Gegenstand der vorliegenden Erfindung ist demgemäss ein Verfahren zur Dehydratisierung von Alkoholen, Polyalkoholen oder Alkoholaten mit mindestens einer CH-Gruppe in α-Stellung zur Alkoholat- bzw. Alkohol-Funktion zu Alkenen , wobei die Dehydratisierung in lonischen Flüssigkeiten der allgemeinen Formel K⁺A⁻ erfolgt gemäβ Anspruch 1. Es hat sich herausgestellt, dass Ionische Flüssigkeiten zur Durchführung der Dehydratisierung in besonderer Weise geeignet sind.

Ionische Flüssigkeiten oder flüssige Salze sind ionische Spezies, die aus einem organischen Kation (K⁺) und einem in der Regel anorganischen Anion (A⁻) bestehen. Sie enthalten keine neutralen Moleküle und weisen meistens Schmelzpunkte kleiner 373 K auf.

Das Gebiet der ionischen Flüssigkeiten wird zur Zeit intensiv erforscht, da die Anwendungsmöglichkeiten vielfältig sind. Übersichtsartikel zu ionischen Flüssigkeiten sind beispielsweise R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Da es sich bei lonischen Flüssigkeiten um Salze handelt, weisen sie keine Flüchtigkeit auf und setzen damit auch keine entzündlichen oder giftigen Dämpfe frei. Sie stellen damit ein sicheres Medium zur Durchführung der Dehydratisierungsreaktion dar. Darüber hinaus hat sich herausgestellt, dass bei Einsatz von lonischen Flüssigkeiten in dem Dehydratisierungsverfahren der sonst übliche Zusatz von Säure zur Katalysierung der Reaktion nicht unbedingt erforderlich ist. Die Ionische Flüssigkeit selbst kann damit die gewünschte Bildung von Alkenen oder Ethern katalysieren. Es hat sich weiterhin herausgestellt, dass die lonischen Flüssigkeiten in der Lage sind, das gebildete Wasser zu binden, so dass eine aufwendige Abtrennung des Wassers vom Alken oder vom Ether umgangen werden kann. Im einfachsten Fall kann das erhaltene Alken oder der Ether von der lonischen Flüssigkeit dekantiert werden und ohne weitere Aufreinigung weiter eingesetzt werden. Auch die Ionische Flüssigkeit lässt sich auf diese Weise einfach recyceln und kann mehrfach wieder verwendet werden. Insgesamt ergibt sich mit dem erfindungsgemäßen Verfahren ein einfacher und kostengünstiger Prozess, der auch für die Alken synthese im kommerziellen Maßstab geeignet ist.

Wesentlich für das erfindungsgemäße Dehydratisierungsverfahren sind die lonischen Flüssigkeiten der allgemeinen Formel K⁺A⁻. Dabei spielt die Auswahl des Anions A⁻ der lonischen Flüssigkeit eine besondere Rolle. Vorzugsweise handelt es sich bei dem Anion A⁻ um ein Anion einer korrespondierenden starken Säure. Insbesondere ist das Anion A⁻ der lonischen Flüssigkeit ausgewählt aus der Gruppe [HSO₄]⁻, [SO₄]²⁻, [NO₃]⁻, [BF₄]⁻, [(R_{F})BF₃]⁻, [(R_{F})₂BF₂]⁻, [(R_{F})₃BF]⁻, [(R_{F})₄B]⁻, [B(CN)₄]⁻, [PO₄]³⁻, [HPO₄]²⁻, [H₂PO₄]⁻, [Alkyl-OPO₃]²⁻, [(Alkyl-O)₂PO₂]⁻, [Alkyl-PO₃]⁻, [R_{F}PO₃]⁻, [(Alkyl)₂PO₂)⁻, [(R_{F})₂PO₂)⁻, [R_{F}SO₃]⁻, [Alkyl-SO₃]⁻, [Aryl-SO₃]⁻, [Alkyl-OSO₃]⁻, [R_{F}C(O)O]⁻, [(R_{F}SO₂)₂N]⁻, {[(R_{F})₂P(O)]₂N}⁻, Cl⁻ und/oder Br⁻
wobei
R_{F} die Bedeutung fluoriertes Alkyl

(CₙF₂ₙ₋ₓ₊₁Hₓ)

mit n = 1-12 und x = 0- 7 hat, wobei für n = 1 x = 0 bis 2 sein soll, und/oder fluoriertes (auch perfluoriertes) Aryl oder Alkyl-Aryl.

Die Alkylgruppe in den oben genannten Anionen kann ausgewählt sein aus geradkettigen oder verzweigten Alkylgruppen mit 1 bis 20 C-Atomen, vorzugsweise mit 1 bis 14 C-Atomen und insbesondere bevorzugt mit 1 bis 4 C-Atomen.

Vorzugsweise bedeutet R_{F} CF₃, C₂F₅, C₃F₇ oder C₄F₉.

In bezug auf die Wahl des Kations K⁺ der lonischen Flüssigkeit der ionischen Flüssigkeit gibt es per se keine Einschränkungen. Vorzugsweise handelt es sich aber um organische Kationen, wobei es sich insbesondere bevorzugt um Ammonium-, Phosphonium, Thiouronium-, Guanidiniumkationen oder um heterocyclische Kationen handelt.

Ammoniumkationen können beispielsweise durch die Formel (1)

[NR₄]⁺ (1),

beschrieben werden, wobei
R jeweils unabhängig voneinander
H, wobei nicht alle Substituenten R gleichzeitig H sein dürfen, geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere R teilweise oder vollständig mit Halogenen, insbesondere -F und/oder -Cl, oder teilweise mit -OR', -CN, - C(O)OH, -C(O)NR'₂, -SO₂NR'₂, -C(O)X, -SO₂OH, -SO₂X, -NO₂, substituiert sein können, und wobei ein oder zwei nicht benachbarte und nicht α-ständige Kohlenstoffatome des R, durch Atome und/oder Atomgruppierungen ausgewählt aus der Gruppe -O-, -S-, -S(O)-, -SO₂-, - N⁺R'₂-, -C(O)NR'-, -SO₂NR'-, -P(O)(NR'₂)NR'-, oder -P(O)R'- ersetzt sein können mit R' = H, nicht, teilweise oder perfluoriertes C₁- bis C₆-Alkyl, C₃-bis C₇-Cycloalkyl, unsubstituiertes oder substituiertes Phenyl und X =Halogen sein kann.

Phosphoniumkationen können beispielsweise durch die Formel (2)

[PR²₄]⁺ (2),

beschrieben werden, wobei
- R²: jeweils unabhängig voneinander
H, NR'₂
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere R² teilweise oder vollständig mit Halogenen, insbesondere -F und/oder -Cl, oder teilweise mit -OR', -CN, - C(O)OH, -C(O)NR'₂, -SO₂NR'₂, -C(O)X, -SO₂OH, -SO₂X, -NO₂, substituiert sein können, und wobei ein oder zwei nicht benachbarte und nicht α-ständige Kohlenstoffatome des R², durch Atome und/oder Atomgruppierungen ausgewählt aus der Gruppe -O-, -S-, -S(O)-, -SO₂-, - N⁺R'₂-, -C(O)NR'-, -SO₂NR'-, -P(O)(NR'₂)NR'- oder -P(O)R'- ersetzt sein können mit R' = H, nicht, teilweise oder perfluoriertes C₁- bis C₆-Alkyl, C₃-bis C₇-Cycloalkyl, unsubstituiertes oder substituiertes Phenyl und X =Halogen.

Ausgeschlossen sind jedoch Kationen der Formeln (1) und (2), in denen alle vier oder drei Substituenten R und R² vollständig mit Halogenen substituiert sind, beispielsweise das Tris(trifluormethyl)methylammonium-kation, das Tetra(trifluormethyl)ammoniumkation oder das Tetra(nonafluorbutyl)ammoniumkation.

Geeignete Thiouroniumkationen können durch die Formel (3),

[(R³R⁴N)-C(=SR⁵)(NR⁶R⁷)]⁺ (3),

beschrieben werden, wobei
- R³ bis R⁷: jeweils unabhängig voneinander
Wasserstoff, wobei Wasserstoff für R⁵ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen, geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R³ bis R⁷ teilweise oder vollständig mit Halogenen, insbesondere -F und/oder -Cl, oder teilweise mit -OH, -OR', -CN, -C(O)OH, -C(O)NR'₂, -SO₂NR'₂, -C(O)X, - SO₂OH, -SO₂X, -NO₂, substituiert sein können, und wobei ein oder zwei nicht benachbarte und nicht α-ständige Kohlenstoffatome von R³ bis R⁷ durch Atome und/oder Atomgruppierungen ausgewählt aus der Gruppe -O-, -S-, -S(O)-, -SO₂-, -SO₂O-, -C(O)O-, -N⁺R'₂-, -P(O)R'O-, -C(O)NR'-, - SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₆-Alkyl, C₃-bis C₇-Cycloalkyl, unsubstituiertes oder substituiertes Phenyl und X =Halogen.

Guanidiniumkationen können durch die Formel (4)

[C(NR⁸R⁹)(NR¹⁰R¹¹)(NR¹²R¹³)] (4),

beschrieben werden, wobei
- R⁸ bis R¹³: jeweils unabhängig voneinander
Wasserstoff, -CN, NR'₂,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R⁸ bis R¹³ teilweise oder vollständig mit Halogenen, insbesondere -F und/oder -Cl, oder teilweise mit -OR', -CN, -C(O)OH, -C(O)NR'₂, -SO₂NR'₂, -C(O)X, -SO₂OH, - SO₂X, -NO₂, substituiert sein können, und wobei ein oder zwei nicht benachbarte und nicht α-ständige Kohlenstoffatome von R⁸ bis R¹³ durch Atome und/oder Atomgruppierungen ausgewählt aus der Gruppe -O-, -S-, - S(O)-, -SO₂-, -N⁺R'₂-, -C(O)NR'-, -SO₂NR'-, -P(O)(NR'₂)NR'- oder -P(O)R'-ersetzt sein können mit R' = H, nicht, teilweise oder perfluoriertes C₁- bis C₆-Alkyl, C₃- bis C₇-Cycloalkyl, unsubstituiertes oder substituiertes Phenyl und X = Halogen.

Darüber hinaus können Kationen der allgemeinen Formel (5)

[HetN]⁺ (5)

eingesetzt werden, wobei
- HetN⁺: ein heterocyclisches Kation, ausgewählt aus der Gruppe 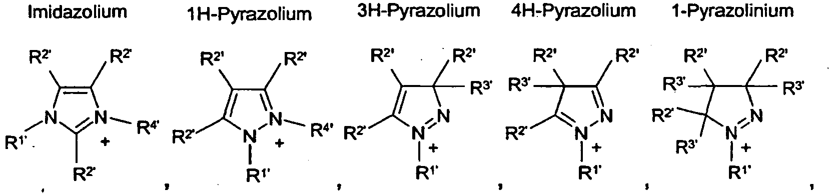 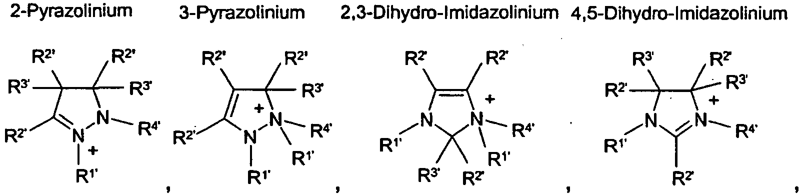 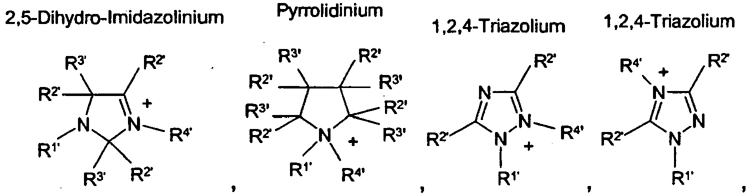 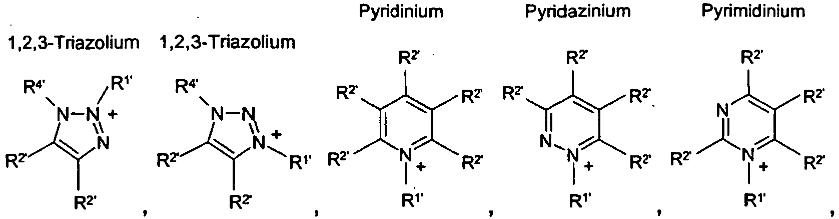 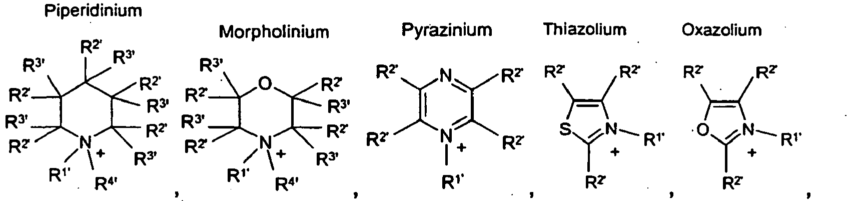 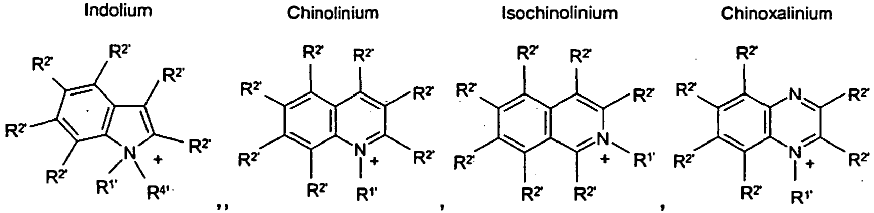 oder 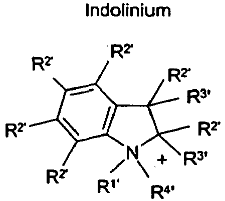
bedeutet, wobei die Substituenten
R^{1,} bis R^{4,} jeweils unabhängig voneinander
Wasserstoff, -CN, -OR', -NR'₂, -P(O)R'₂, -P(O)(NR'₂)₂, -C(O)R', geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl bedeutet,
wobei die Substituenten R^{1'}, R^{2'}, R^{3'} und/oder R^{4'} zusammen auch ein Ringsystem bilden können,
wobei ein oder mehrere Substituenten R^{1,} bis R^{4,} teilweise oder vollständig mit Halogenen, insbesondere -F und/oder -Cl, oder -OR', -CN, -C(O)OH, - C(O)NR'₂, -SO₂NR'₂, -C(O)X, -SO₂OH, -SO₂X, -NO₂, substituiert sein können, wobei jedoch nicht gleichzeitig R¹' und R^{4'} vollständig mit Halogenen substituiert sein dürfen, und wobei ein oder zwei nicht benachbarte und nicht am Heteroatom gebundene Kohlenstoffatome der Substituenten R^{1,} bis R^{4,} durch Atome und/oder Atomgruppierungen ausgewählt aus der -O-, -S-, -S(O)-, -SO₂-, -C(O)-, -N⁺R'₂-, -C(O)NR'-, - SO₂NR'-, -P(O)(NR'₂)NR'-, -PR'₂=N- oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen.

Unter vollständig ungesättigten Substituenten werden im Sinne der vorliegenden Erfindung auch aromatische Substituenten verstanden.

Als Substituenten R und R² bis R¹³ der Verbindungen der Formeln (1) bis (5) kommen erfindungsgemäß dabei neben Wasserstoff bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R und R² in den Verbindungen der Formel (1) oder (2) können dabei gleich oder verschieden sein. Bevorzugt sind die Substituenten R und R² verschieden.

Die Substituenten R und R² sind insbesondere bevorzugt Methyl, Ethyl, Isopropyl, Propyl, Butyl, sek.-Butyl, tert.-Butyl, Pentyl, Hexyl, Octyl, Decyl oder Tetradecyl.

Bis zu vier Substituenten des Guanidinium-Kations [C(NR⁸R⁹)(NR¹⁰R¹¹)(NR¹²R¹³)]⁺ können auch paarweise derart verbunden sein, dass mono-, bi- oder polycyclische Kationen entstehen.

Ohne Einschränkung der Allgemeinheit sind Beispiele für solche Guanidinium-Kationen: 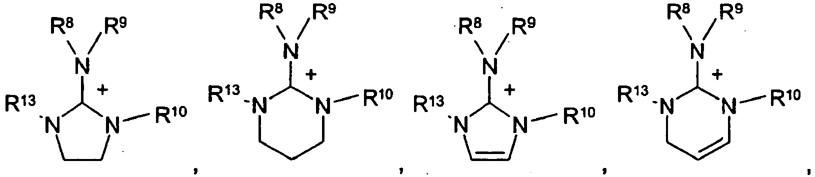 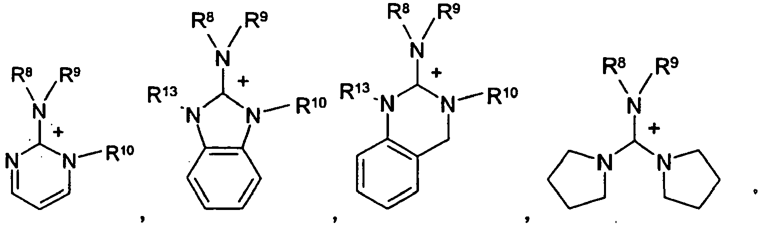 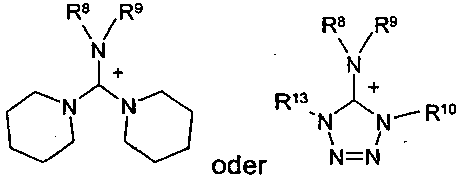 wobei die Substituenten R⁸ bis R¹⁰ und R¹³ eine zuvor angegebene oder besonders bevorzugte Bedeutung haben können.
Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CF₃, COOH, SO₂NR'₂, SO₂X' oder SO₃H substituiert sein, wobei X und R' eine zuvor angegebene Bedeutung haben, substituiertes oder unsubstituiertes Phenyl oder unsubstituierter oder substituierter Heterocyclus substituiert sein.

Bis zu vier Substituenten des Thiouroniumkations [(R³R⁴N)-C(=SR⁵)(NR⁶R⁷)]⁺ können auch paarweise derart verbunden sein, dass mono-, bi- oder polycyclische Kationen entstehen.

Ohne Einschränkung der Allgemeinheit sind Beispiele für solche Kationen im folgenden angegeben, wobei Y = S bedeutet: 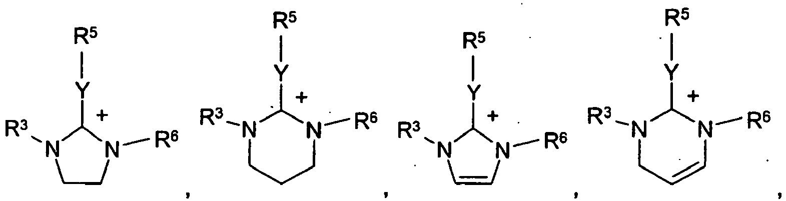 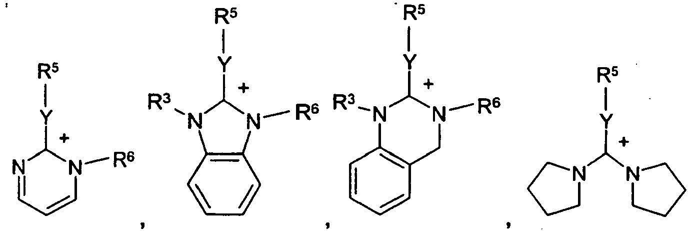 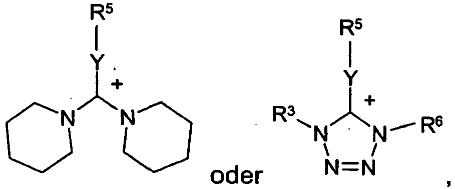 wobei die Substituenten R³, R⁵ und R⁶ eine zuvor angegebene oder besonders bevorzugte Bedeutung haben können.
Gegebenenfalls können die Carbocyclen oder Heterocyclen der zuvor angegebenen Kationen noch durch C₁- bis C₆-Alkyl, C₁- bis C₆-Alkenyl, NO₂, F, Cl, Br, I, C₁-C₆-Alkoxy, SCF₃, SO₂CF₃, COOH, SO₂NR'₂, SO₂X oder SO₃H oder substituiertes oder unsubstituiertes Phenyl oder unsubstituierter oder substituierter Heterocyclus substituiert sein, wobei X und R' eine zuvor angegebene Bedeutung haben.

Die Substituenten R³ bis R¹³ sind jeweils unabhängig voneinander bevorzugt eine geradkettige oder verzweigte Alkylgruppe mit 1 bis 10 C-Atomen. Die Substituenten R³ und R⁴, R⁶ und R⁷, R⁸ und R⁹, R¹⁰ und R¹¹ und R¹² und R¹³ in Verbindungen der Formeln (3) bis (5) können dabei gleich oder verschieden sein. Besonders bevorzugt sind R³ bis R¹³ jeweils unabhängig voneinander Methyl, Ethyl, n-Propyl, Isopropyl, n-Butyl, tert.-Butyl, sek.-Butyl, Phenyl oder Cyclohexyl, ganz besonders bevorzugt Methyl, Ethyl, n-Propyl, Isopropyl oder n-Butyl.

Als Substituenten R^{1'} bis R^{4'} von Verbindungen der Formel (5) kommen erfindungsgemäß dabei neben Wasserstoff bevorzugt in Frage: C₁- bis C₂₀, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R^{1'} und R^{4'} sind jeweils unabhängig voneinander insbesondere bevorzugt Methyl, Ethyl, Isopropyl, Propyl, Butyl, sek.-Butyl, tert.-Butyl, Pentyl, Hexyl, Octyl, Decyl, Cyclohexyl, Phenyl oder Benzyl. Sie sind ganz besonders bevorzugt Methyl, Ethyl, n-Butyl oder Hexyl. In Pyrrolidinium-, Piperidinium- oder Indolinium-Verbindungen sind die beiden Substituenten R^{1'} und R^{4'} bevorzugt unterschiedlich.

Der Substituent R^{2'} oder R^{3'} ist jeweils unabhängig voneinander insbesondere Wasserstoff, Methyl, Ethyl, Isopropyl, Propyl, Butyl, sek.-Butyl, tert.-Butyl, Cyclohexyl, Phenyl oder Benzyl. Besonders bevorzugt ist R^{2'} Wasserstoff, Methyl, Ethyl, Isopropyl, Propyl, Butyl oder sek.-Butyl. Ganz besonders bevorzugt sind R^{2'} und R^{3'} Wasserstoff.
Die C₁-C₁₂-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl oder Dodecyl. Gegebenenfalls Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis **-C₂₀H₃₇,** vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilweise mit -OR', -CN, -C(O)OH, -C(O)NR'₂, -SO₂NR'₂, -C(O)X, -SO₂OH, -SO₂X, -NO₂ substituiert sein können.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl oder mit -OR', -CN, -C(O)OH, - C(O)NR'₂, -SO₂NR'₂, -C(O)X, -SO₂OH, -SO₂X, -NO₂ substituiert sein kann.

In den Substituenten R, R² bis R¹³ oder R^{1'} bis R^{4'} können auch ein oder zwei nicht benachbarte und nicht α-ständig zum Heteroatom gebundene Kohlenstoffatome, durch Atome und/oder Atomgruppierungen ausgewählt aus der Gruppe -O-, -S-, -S(O)-, -SO₂-, -N⁺R'₂-, -C(O)NR'-, -SO₂NR'-, - P(O)(NR'₂)NR'- oder -P(O)R'- ersetzt werden, mit R' = nicht, teilweise oder perfluoriertes C₁- bis C₆-Alkyl, C₃- bis C₇-Cycloalkyl, unsubstituiertes oder substituiertes Phenyl.

Ohne Einschränkung der Allgemeinheit sind Beispiele für derart modifizierte Substituenten R, R² bis R¹³ und R¹' bis R^{4'}:
-OCH₃, -OCH(CH₃)₂, -CH₂OCH₃, -CH₂-CH₂-O-CH₃, -C₂H₄OCH(CH₃)₂, - C₂H₄SC₂H₅, -C₂H₄SCH(CH₃)₂, -S(O)CH₃, -SO₂CH₃, -SO₂C₆H₅, -SO₂C₃H₇, - SO₂CH(CH₃)₂, -SO₂CH₂CF₃, -CH₂SO₂CH₃, -O-C₄H₈-O-C₄H₉, -CF₃, -C₂F₅, - C₃F₇, -C₄F₉, -C(CF₃)₃, -CF₂SO₂CF₃, -C₂F₄N(C₂F₅)C₂F₅, -CHF₂, -CH₂CF₃, - C₂F₂H₃, -C₃FH₆, -CH₂C₃F₇, -C(CFH₂)₃, -CH₂C(O)OH, -CH₂C₆H₅ oder P(O)(C₂H₅)₂.

In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, C₁-C₆-Alkoxy, SCF₃, SO₂CF₃, COOH, SO₂X', SO₂NR"₂ oder SO₃H substituiertes Phenyl, wobei X' F, Cl oder Br und R" ein nicht, teilweise oder perfluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m-oder p-Isopropylphenyl, o-, m- oder p-tert.-Butylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

In R^{1'} bis R^{4'} wird als Heteroaryl ein gesättigter oder ungesättigter monooder bicyclischer heterocyclischer Rest mit 5 bis 13 Ringgliedern verstanden, wobei 1, 2 oder 3 N- und/oder 1 oder 2 S- oder O-Atome vorliegen können und der heterocyclische Rest ein- oder mehrfach durch C₁- bis C₆-Alkyl, C₁- bis C₆-Alkenyl, NO₂, F, Cl, Br, I, C₁-C₆-Alkoxy, SCF₃, SO₂CF₃, COOH, SO₂X', SO₂NR"₂ oder SO₃H substituiert sein kann, wobei X' und R" eine zuvor angegebene Bedeutung haben.
Der heterocyclische Rest ist vorzugsweise substituiertes oder unsubstituiertes 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-lsoxazolyl, 2-, 4- oder 5-Thiazolyl, 3-, 4- oder 5-Isothiazolyl, 2-, 3-oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1 H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4-oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7- oder 8-Chinazolinyl oder 1-, 2- oder 3-Pyrrolidinyl.

Unter Heteroaryl-C₁-C₆-alkyl wird nun in Analogie zu Aryl-C₁-C₆-alkyl beispielsweise Pyridinyl-methyl, Pyridinyl-ethyl, Pyridinyl-propyl, Pyridinylbutyl, Pyridinyl-pentyl, Pyridinyl-hexyl verstanden, wobei weiterhin die zuvor beschriebenen Heterocyclen in dieser Weise mit der Alkylenkette verknüpft werden können.

HetN⁺ ist bevorzugt 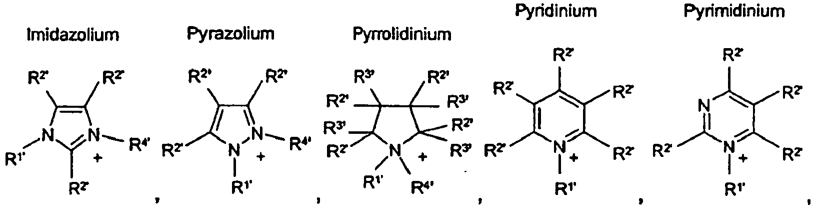 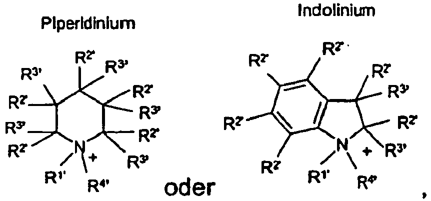 wobei die Substituenten R^{1'} bis R^{4'} jeweils unabhängig voneinander eine zuvor beschriebene Bedeutung haben.

Vorzugsweise handelt es sich bei den Kationen der erfindungsgemäßen ionischen Flüssigkeit um Ammonium-, Phosphonium-, Imidazolium-, Pyridinium- oder Pyrrolidinium-Kationen.

Besonders bevorzugte lonische Flüssigkeiten sind Ammonium-, Phosphonium-, Imidazolium- oder Pyrrolidinium-Hydrogensulfate, - Alkylsulfate, -Alkylsulfonate, -Perfluoralkylsulfonate, -Phosphate, - Hydrogenphosphate, -Alkylphosphate, -Alkyl und Perfluoralkyl-phosphinate, -Alkyl und Perfluoroalkyl-phosphonate oder -Perfluoralkylcorboxylate.

Die Verfahrenstemperatur ist an sich nicht kritisch und beträgt üblicherweise 0 bis 170°C, vorzugsweise 20 bis 120°C.

Insgesamt sind daher mit dem neuen erfindungsgemäßen Verfahren Wasserabspaltungen aus Alkoholaten bzw. Alkoholen oder Polyalkoholen zugänglich, die mit bekannten Verfahren nicht durchführbar waren, und dabei sind die Dehydratisierungsreaktionen deutlich besser optimierbar. In dem beschriebenen System ist die Wasserabspaltung aus Alkoholen in zweierlei Weise möglich; intramolekular und zwischenmolekular. Im ersten Fall bilden sich Alkene wobei im zweiten Fall Ether, z.B. Dialkylether resultieren. Vorzugsweise werden mit dem erfindungsgemäßen Verfahren Alkene hergestellt.

Die erfindungsgemäßen Verfahren sind geeignet für die Wasserabspaltung nicht nur aus Alkoholen, sondern auch aus Polyalkoholen, z.B. Glykolen, Triolen oder natürlichen Stoffen, wie z.B. Polysaccariden, Hexosen oder Pentosen.

Besonders vorteilhaft wird das erfindungsgemäße Verfahren zur Synthese von arylsubstituierten Alkenen eingesetzt, die z.B als mesogene Substanzen, Pharmawirkstoffe, Pflanzenschutzmittel, Polymere oder Vorstufen in der Feinchemie oder zur Herstellung entsprechender Ausgangsverbindungen Verwendung finden.

Vorzugsweise wird als Alkoholat bzw. Alkohol, im folgenden gemeinsam als Alkoholat bezeichnet, eine Verbindung der Formel verwendet 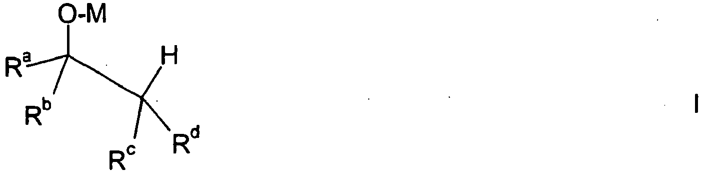 in der
- M: ein Alkalimetall, ein Erdalkalimetallhalogenid oder ein H-Atom bedeutet und
- R^{b}, R^{c}, R^{d}: unabhängig voneinander einen ggf. substituierten aliphatischen oder aromatischen Rest, der ein oder mehrere Heteroatome aufweisen kann, bedeuten, wobei ein, zwei oder drei Reste der Gruppe R^{a} bis R^{d} auch H bedeuten können, und wobei zwei oder mehr der Reste R^{a}, R^{b}, R^{c} und/oder R^{d} miteinander verbunden sein können,
- R^{a}: eine Bedentung der Formel Ia aufweist

R^{e}(A⁰Z⁰)ₚ(A¹)ᵣ la worin

R^{e} einen unsubstituierten, einen einfach oder mehrfach durch CN und/oder Halogen substituierten Alkylrest mit 1 bis 15 C-Atomen, wobei in diesen Resten auch eine oder mehrere CH₂-Gruppen durch -O-, -S-, -CH=CH-, -C≡C-, -OC-O- und/oder-O-CO-, und/oder auch eine oder mehrere CH-Gruppen durch N oder P so ersetzt sein können, dass zwei O-Atome nicht direkt miteinander verknüpft sind, oder, falls r und/oder p verschieden von 0 sind, auch H, Halogen, CN, SF₅ oder NCS,
A⁰, A¹ jeweils unabhängig voneinander
a) einen 1,4-Cyclohexenylen- oder 1,4-Cyclohexylenrest, worin eine oder zwei nicht benachbarte CH₂-Gruppen durch -O-oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl, 1,4-Bicyclo[2,2,2]octylen, Phenanthren-2,7-diyl, Naphthalin-2,6-diyl, Decahydronaphthalin-2,6-diyl, und 1,2,3,4-Tetrahydronaphthalin-2,6-diyl,
d) einen bivalenten Rest aus der Gruppe Furan, Pyrrol, Thiophen, Pyrazol, Imidazol, 1,2-Oxazol, 1,3-Oxazol, Thiazol, Pyridin, Pyridazin, Pyrimidin, Pyrazin, 2H-Pyran, 4H-Pyran, Purin, Pteridin, 1H-Azepin, 3H-1,4-Diazepin, Indol, Benzofuran, Benzothiophen, Chinolin, Isochinolin, Phenazin, Phenoxazin, Phenothiazin und 1,4-Benzodiazepin, wobei die Reste a), b), c) und d) ein- oder mehrfach durch R^{e}, insbesondere durch Halogen und/oder CN, substituiert sein können,
Z⁰ -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- oder eine Einfachbindung,
p 0, 1, 2 oder 3 und
r 1 oder 2 bedeutet.

Vorzugsweise sind die Reste R^{a} und R^{b} und/oder R^{b} und R^{c} miteinander verbunden, beispielsweise unter Ausbildung eines aliphatischen und/oder aromatischen Rings oder anellierten Ringsystems, der bzw. das auch ein oder mehrere Heteroatome aufweisen kann.

Bevorzugte Bedeutungen von M sind H, Li, MgCl, MgBr oder Mgl.

Bevorzugte Bedeutungen von R^{e} sind geradkettige oder verzweigte Alkylund Alkoxyreste mit 1 bis 8C-Atomen, die einfach durch -CN und/oder einoder mehrfach durch Halogen substituiert sein können.

Bevorzugte Bedeutungen von A⁰ und/oder A¹ sind 1,4-Cyclohexylen, worin eine oder zwei nicht benachbarte CH₂-Gruppen durch -0- ersetzt sein können, 1,4-Phenylen, worin eine oder zwei CH-Gruppen durch N ersetzt sein können, Phenanthren-2,7-diyl-, Naphthalin-2,6-diyl, Decahydronaphthalin-2,6-diyl, und 1,2,3,4-Tetrahydronaphthalin-2,6-diyl, wobei diese Reste ein- oder mehrfach durch Halogen, insbesondere Fluor und/oder Chlor, CN und/oder gegebenenfalls durch Halogen substituiertes C₁₋₅-Alkyl oder -Alkoxy substituiert sein können.

Besonders bevorzugt ist A¹ eine 1,4-Phenylen-Gruppe, die unsubstituiert oder in 2, 3, 5 und/oder 6-Position ein-, zwei-, drei- oder vierfach mit Fluor substituiert ist, womit die erfindungsgemäße Umsetzung nach folgendem Schema verläuft: 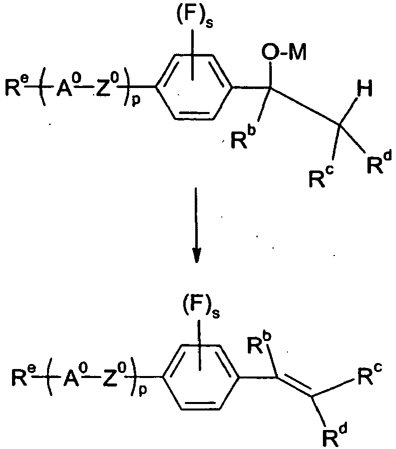 worin R^{e}, A⁰, Z⁰, p, M, R^{b}, R^{c} und R^{d} die vor- und nachstehend angegebenen Bedeutungen aufweisen und s 0, 1, 2, 3 oder 4 bedeutet.

Bevorzugt sind:
- p = r = 0, wobei R^{e} vorzugsweise ein geradkettiger oder verzweigter Alkylrest mit 1 bis 8 C-Atomen ist, der einfach durch -CN und/oder einoder mehrfach durch Halogen substituiert sein kann,
- r = 1 und p = 0, 1 oder 2.

R^{a} kann auch Bestandteil eines Liganden, beispielsweise eines Cyclopentadienyl-Systems in einem metallorganischen Komplex, sein.

Besonders bevorzugte Gruppen R^{a} sind nachfolgend aufgeführt: 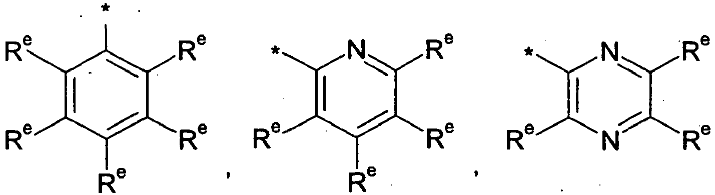 und 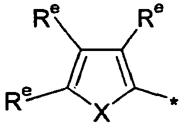 worin X O, NR^{e} oder S bedeutet, R^{e} die angegebene Bedeutung besitzt und * die freie Bindung angibt.

Vor- und nachstehend bedeutet Halogen als Substituent organischer Reste Fluor, Chlor, Brom oder lod, vorzugsweise Fluor oder Chlor, besonders bevorzugt Fluor.

Vor- und nachstehend können mehrfach vorkommende Gruppen und Substituenten, wie beispielsweise A⁰, Z⁰, A¹, R^{e}, jeweils gleiche oder unterschiedliche Bedeutungen aufweisen.

Vorzugsweise besitzen ein, zwei oder drei Reste der Gruppe R^{b}, R^{c}, R^{d} unabhängig voneinander eine Bedeutung gemäß der Formel la und die gegebenenfalls übrigen Reste der Gruppe R^{b}, R^{c}, R^{d} bedeuten H.

Besonders bevorzugt ist R^{d} H und R^{b} und/oder R^{c} besitzen eine von H verschiedene Bedeutung. Ganz besonders bevorzugt sind R^{b} und R^{c} verschieden von H.

Weiterhin bevorzugt sind R^{b} und R^{c} derart miteinander verbunden, dass das Alkoholat der Formel I eine Bedeutung gemäß Formel lb besitzt 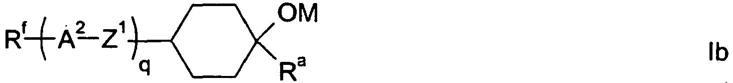 worin
- R^{f}: eine der zu R^{e} angegebenen Bedeutungen besitzt,
- A²: eine der zu A⁰, A¹ angegebenen Bedeutungen besitzt,
- Z¹: eine der zu Z⁰ angegebenen Bedeutungen besitzt,
- q: 0, 1, 2 oder 3 bedeutet und

R^{a} und M die zuvor und nachfolgend angegebenen Bedeutungen besitzen.

Die Alkoholate der Formel I sind in guten bis sehr guten Ausbeuten durch Addition von metallorganischen Verbindungen an Verbindungen mit einer oder mehreren Carbonylfunktionen erhältlich. Solche Umsetzungen sowie die einzusetzenden Edukte, Lösungsmittel und Reaktionsbedingungen sind dem Fachmann bekannt oder in Abwandlung bekannter Synthesen ohne weiteres zu erhalten.

Es versteht sich für den Fachmann von selbst, dass in den genannten lonischen Flüssigkeiten oder Alkoholen oder Alkoholaten Substituenten wie beispielsweise H, N, O, Cl, F durch die entsprechenden Isotope ersetzt sein können.

Die folgenden Ausführungsbeispiele sollen die Erfindung erläutern, ohne sie zu begrenzen. Vor- und nachstehend bedeuten Prozentangaben Gewichtsprozent. Alle Temperaturen sind in Grad Celsius angegeben.

### Beispiele:

### Beispiel 1: Synthese von 1-Phenyl-cyclohex-1-en

Zu 10 ml Ethyl-Methyl-imidazoliumhydrogensulfat wird 1-Phenyl-1-cyclohexanol zugegeben und die Mischung wird bei 80-90°C für eine Stunde gerührt. Nach Abkühlung bilden sich zwei Phasen, wobei die obere Phase, die Produktphase, abdekantiert wird. Die untere Phase, die Ionische Flüssigkeit, wird erneut mit 1-Phenyl-1-cyclohexanol versetzt und entsprechend umgesetzt und abgetrennt. Der genannte Vorgang kann mehrfach wiederholt werden, ohne die ionische Flüssigkeit zu wechseln. Die mittlere Ausbeute an 1-Phenyl-cyclohex-1-en beträgt 97.2%, das Produkt kann durch Destillation weiter aufgereinigt werden.
Das isolierte Produkt wird mittels NMR-Spektroskopie untersucht.
¹H NMR (Referenz: TMS; Lösungsmittel: CD₃CN), ppm: 1.90 m (CH₂); 2.01 m, (CH₂); 2.43 m (CH₂); 2.64 m (CH₂); 6.36 m (1CH); 7.54 m (5CH, Ph).

### Referenz beispiel 2: Synthese von isobutylen

Eine Mischung aus Ethyl-Methyl-imidazoliumhydrogensulfat und konzentrierter Schwefelsäure (Volumenverhältnis 3.75:1) wird mit tert-Butanol versetzt. Die Reaktions-Mischung (eine Emulsion) wird 4 Stunden bei 43°C gerührt. Entstandenes Isobutylen wird bei -196°C (flüssiger Stickstoff) und Normaldruck in eine Falle kondensiert. Die Falle wird anschließend bis -78° C erwärmt, abgeschmolzen und bei Raumtemperatur gewogen. Es wird Isobutylen als klare und farblose Flüssigkeit isoliert. Der genannte Vorgang kann mehrfach wiederholt werden, ohne die ionische Flüssigkeit zu wechseln.
Die durchschnittliche Ausbeute an isoliertem Isobutylen beträgt 92%. Das isolierte Produkt wird mittels NMR-Spektroskopie untersucht.
¹H NMR (Referenz: TMS; Lösungsmittel: CDCl₃), ppm: 1.55 t (2CH₃); 4.49 sep, (CH₂); ⁴JH,H = 1.1 Hz.

### Referenz beispiel 3: Synthese von Cyclohexen

Zu einer Mischung aus Ethyl-Methyl-imidazoliumhydrogensulfat und Cyclohexanol wird konzentrierte Schwefelsäure (97-98%) zugegeben (Volumenverhältnis 1.7:2:1). Nach stark exothermer Reaktion und intensivem Rühren homogenisiert die Emulsion. Die entstandene Lösung wird eine Stunde bei 75°C gerührt und gebildetes Cyclohexen wird abdestilliert. Die Ausbeute an Cyclohexen beträgt 82%.
Das isolierte Produkt wird mittels NMR-Spektroskopie untersucht.
¹H NMR (Referenz: TMS; Lösungsmittel: CDDl₃), ppm: 1.50 m (2CH₂); 1.87 m, (2CH₂); 5.53 m (2CH).

### Referenz beispiel 4: Synthese von 2,3-Dimethylbuta-1,3-dien

Eine Mischung aus Ethyl-Methyl-imidazoliumhydrogensulfat und 2,3-Dimethyl-2,3-butandiol (Massenverhältnis 1:1) wird auf 140°C erhitzt und gebildetes 2,3-Dimethylbuta-1,3-dien zusammen mit anderen Dehydratisierungsprodukten (wie z.B. 2,3-Epoxy-2,3-dimethyl-butan) unter Normaldruck abdestilliert. Das reine 2,3-Dimethylbuta-1,3-dien kann durch anschließende fraktionierte Destillation isoliert werden. Die Ausbeute an isoliertem 2,3-Dimethylbuta-1,3-dien beträgt 60%. Der genannte Vorgang kann mehrfach wiederholt werden, ohne die ionische Flüssigkeit zu wechseln.
Das isolierte Produkt wird mittels NMR-Spektroskopie untersucht.
¹H NMR (Referenz: TMS; Lösungsmittel: CDCl₃), ppm: 1.68 s (2CH₃); 4.73 m, (2CH); 4.82 m (2CH).

### Referenz beispiel 5: Synthese von Diheptyelther

Zu einer Mischung aus Ethyl-Methyl-imidazoliumhydrogensulfat und 1-Heptanol wird konzentrierte Schwefelsäure (97-98%) zugegeben (Volumenverhältnis 2:1.3:1). Nach exothermer Reaktion und intensivem Rühren homogenisiert die Emulsion. Die entstandene Lösung wird 2.5 Stunden bei 117°C gerührt und gebildeter Diheptylether wird extrahiert und durch fraktionierte Destillation isoliert. Die Ausbeute an isoliertem Diheptylether beträgt 50%.
Das isolierte Produkt wird mittels NMR-Spektroskopie untersucht.
¹H NMR (Referenz: TMS; Lösungsmittel: CD₃CN), ppm: 0.85 m (2CH₂); 1.27 m, (8CH₂); 1.30 m (2CH₂); 3.70 t (2CH₂); ³J_{H,H} = 6.8 Hz.
¹³C {¹H} NMR (Referenz: TMS; Lösungsmittel: CD₃CN), ppm: 14.1 s; 23.0 s; 25.9 s; 29.1 s; 29.4 s; 32.0 s; 70.1 s.

### Beispiel 6: Mischungen von 1-Butyl-3-methylimidazoliumtrifluoracetat mit Trifluoressigsäure.

Zu 1-Butyl-3-methylimidazoliumtrifluoroacetat werden 10 Gewicht-% (bzw. 20 Gewichts-%) Trifluoressigsäure zugegeben. Das resultierende Gemisch wird mittels TGA Methode untersucht.
Das Gemisch mit 20 Gew.-% Trifluor-essigsäure (Siedepunkt von freier Säure beträgt 72-73°C) weist bei 140°C nur einen Gewichtsverlust von ca. 2.5 % auf.
Das Gemisch mit 10 Gew.-% Trifluor-essigsäure Trifluoressigsäure weist bei 140°C nur einen Gewichtsverlust von weniger als 2 % Verlust auf.

## Patentansprüche

1. Verfahren zur Dehydratisierung von Polyalkoholen, Alkoholen oder Alkoholaten mit mindestens einer CH-Gruppe in α-Stellung zur Alkoholat- bzw. Alkohol-Funktion zu Alkenen, wobei der Alkohol ausgewählt ist aus Verbindungen der Formel (I) 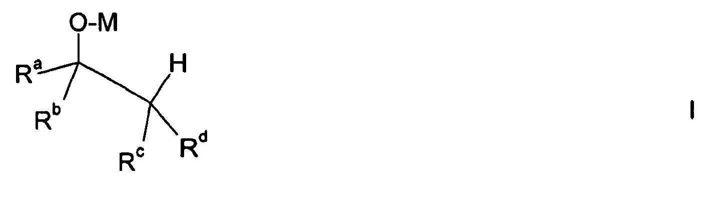 wobei
M ein Alkalimetall, ein Erdalkalimetallhalogenid oder ein H-Atom bedeutet und
R^{b}, R^{c}, R^{d} unabhängig voneinander einen ggf. substituierten aliphatischen oder aromatischen Rest, der ein oder mehrere Heteroatome aufweisen kann, bedeuten, wobei ein, zwei oder drei Reste der Gruppe R^{a} bis R^{d} auch H bedeuten können, und wobei zwei oder mehr der Reste R^{a}, R^{b}, R^{c} und/oder R^{d} miteinander verbunden sein können,
R^{a} eine Bedeutung der Formel la aufweist 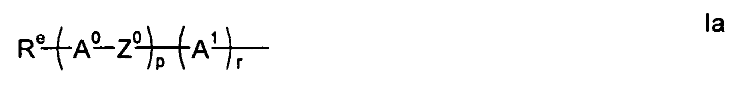 worin
R^{e} einen unsubstituierten, einen einfach oder mehrfach durch CN und/oder Halogen substituierten Alkylrest mit 1 bis 15 C-Atomen, wobei in diesen Resten auch eine oder mehrere CH₂-Gruppen durch -O-, -S-, -CH=CH-, -C≡C-, -OC-O- und/oder -O-CO-, und/oder auch eine oder mehrere CH-Gruppen durch N oder P so ersetzt sein können, dass zwei O-Atome nicht direkt miteinander verknüpft sind, oder, falls r und/oder p verschieden von 0 sind, auch H, Halogen, CN, SF₅ oder NCS,
A⁰, A¹ jeweils unabhängig voneinander
a) einen 1,4-Cyclohexenylen- oder 1,4-Cyclohexylenrest, worin eine oder zwei nicht benachbarte CH₂-Gruppen durch -O- oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl, 1,4-Bicyclo[2,2,2]octylen, Phenanthren-2,7-diyl, Naphthalin-2,6-diyl, Decahydronaphthalin-2,6-diyl, und 1,2,3,4-Tetrahydronaphthalin-2,6-diyl,
d) einen bivalenten Rest aus der Gruppe Furan, Pyrrol, Thiophen, Pyrazol, Imidazol, 1,2-Oxazol, 1,3-Oxazol, Thiazol, Pyridin, Pyridazin, Pyrimidin, Pyrazin, 2H-Pyran, 4H-Pyran, Purin, Pteridin, 1 H-Azepin, 3H-1,4-Diazepin, Indol, Benzofuran, Benzothiophen, Chinolin, Isochinolin, Phenazin, Phenoxazin, Phenothiazin und 1,4-Benzodiazepin,
wobei die Reste a), b), c) und d) ein oder mehrfach durch R^{e}, insbesondere durch Halogen und/oder CN, substituiert sein können,
Z⁰ -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- oder eine Einfachbindung,
p 0, 1, 2 oder 3 und
r 1 oder 2 bedeutet, **dadurch gekennzeichnet, dass** die Dehydratisierung in lonischen Flüssigkeiten der allgemeinen Formel K⁺A⁻ erfolgt, wobei das Anion A⁻ der lonischen Flüssigkeit ausgewählt ist aus der Gruppe [HSO₄]⁻, [SO₄]²⁻, [NO₃]⁻, [BF₄]⁻, [(R_{F})BF₃]⁻, [(R_{F})₂BF₂]⁻, [(R_{F})₃BF]⁻, [(R_{F})₄B]⁻, [B(CN)₄]⁻, [PO₄]³⁻, [HPO₄]²⁻, [H₂PO₄]⁻, [Alkyl-OPO₃]²⁻, [(Alkyl-O)₂PO₂]⁻, [Alkyl-PO₃]⁻, [R_{F}PO₃]⁻, [(Alkyl)₂PO₂]⁻, [(R_{F})₂PO₂]⁻, [R_{F}SO₃]⁻, [Alkyl-SO₃]⁻, [Aryl-SO₃]⁻, [Alkyl-OSO₃]⁻, [R_{F}C(O)O]⁻, [(R_{F}SO₂)₂N]⁻, {[(R_{F})₂P(O)]₂N}⁻, Cl⁻ und/oder Br⁻, wobei R_{F} die Bedeutung fluoriertes Alkyl
(CₙF₂ₙ₋ₓ₊₁ Hₓ)
mit n = 1-12 und x = 0- 7 hat, wobei für n = 1 x = 0 bis 2 sein soll, und/oder fluoriertes Aryl oder Alkyl-Aryl hat und die Kationen ausgewählt sind aus der Gruppe der Ammonium-, Phosphonium-, Thiouronium-, Guanidiniumkationen oder heterocyclischen Kationen

2. Verfahren nach Anspruch 1, **dadurch gekennzeichnet, dass** die Ammoniumkationen der Formel (1) entsprechen,
[NR₄]⁺ (1),
beschrieben werden, wobei
R jeweils unabhängig voneinander
H, wobei nicht alle Substituenten R gleichzeitig H sein dürfen, geradkettiges oder verzweigtes Alkyl mit 1-20 C-Atomen oder gesättigtes oder ungesättigtes C₃- bis C₇-Cycloalkyl, das mit C₁- bis C₆-Alkylgruppen substituiert sein kann bedeutet oder dass die Phosphoniumkationen der Formel (2) entsprechen,
[PR²₄]⁺ (2),
wobei
R² jeweils unabhängig voneinander
H,
geradkettiges oder verzweigtes Alkyl mit 1-20 C-Atomen oder gesättigtes oder ungesättigtes C₃- bis C₇-Cycloalkyl, das mit C₁- bis C₆-Alkylgruppen substituiert sein kann bedeutet oder dass die Thiouroniumkationen der Formel (3) entsprechen
[(R³R⁴N)-C(=SR⁵)(NR⁶R⁷)]⁺ (3),
wobei
R³ bis R⁷ jeweils unabhängig voneinander
Wasserstoff, wobei Wasserstoff für R⁵ ausgeschlossen wird, geradkettiges oder verzweigtes Alkyl mit 1 bis 20 C-Atomen oder gesättigtes oder ungesättigtes C₃- bis C₇-Cycloalkyl, das mit C₁- bis C₆-Alkylgruppen substituiert sein kann bedeuten oder dass die Guanidiniumkationen der Formel (4) entsprechen
[C(NR⁸R⁹)(NR¹⁰R¹¹)(NR¹²R¹³)]⁺ (4),
wobei
R⁸ bis R¹³ jeweils unabhängig voneinander
Wasserstoff,
geradkettiges oder verzweigtes Alkyl mit 1 bis 20 C-Atomen oder gesättigtes oder ungesättigtes C₃- bis C₇-Cycloalkyl, das mit C₁- bis C₆-Alkylgruppen substituiert sein kann bedeuten oder
dass die heterocyclischen Kationen der Formel (5) entsprechen,
[HetN]⁺ (5),
wobei
HetN⁺ ein heterocyclisches Kation, ausgewählt aus der Gruppe 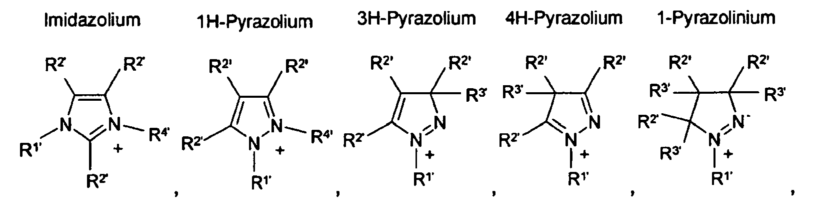 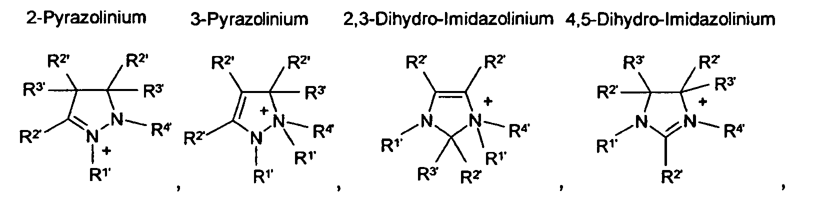 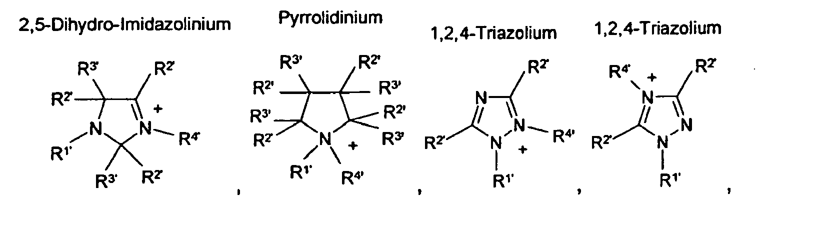 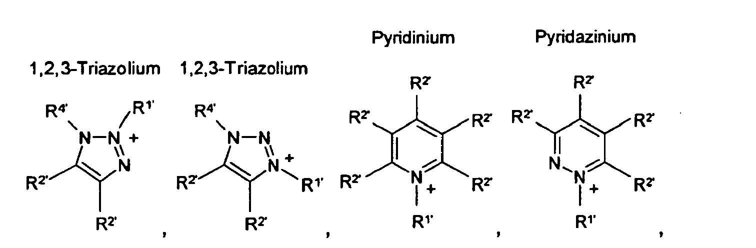 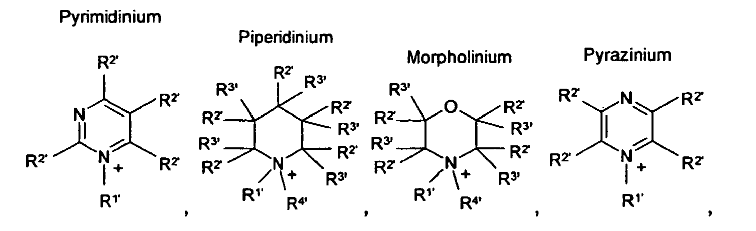 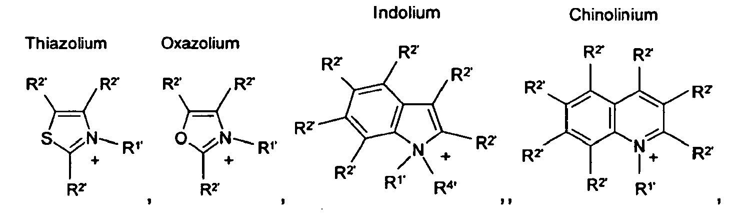 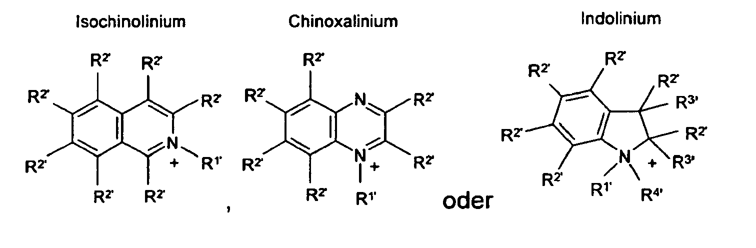 bedeutet, wobei die Substituenten
R¹_{,} bis R^{4,} jeweils unabhängig voneinander
Wasserstoff,
geradkettiges oder verzweigtes Alkyl mit 1-20 C-Atomen oder oder gesättigtes oder ungesättigtes C₃- bis C₇-Cycloalkyl, das mit C₁- bis C₆-Alkylgruppen substituiert sein kann bedeuten.

3. Verfahren nach Anspruch 1 oder 2, **dadurch gekennzeichnet, dass** die Verfahrenstemperatur 0 bis 170°C beträgt.

4. Verfahren gemäß Anspruch 1, **dadurch gekennzeichnet, dass** M H, Li, MgCl, MgBr oder Mgl bedeutet.

5. Verfahren nach einem der vorherigen Ansprüche, **dadurch gekennzeichnet, dass** ein, zwei oder drei Reste der Gruppe R^{b}, R^{c}, R^{d} unabhängig voneinander eine Bedeutung gemäß der Formel la besitzen und die gegebenenfalls übrigen Reste der Gruppe R^{b}, R^{c}, R^{d} H bedeuten.

## Claims

1. Process for the dehydration of polyalcohols, alcohols or alcoholates having at least one CH group in the α-position to the alcoholate or alcohol function to give alkenes, where the alcohol is selected from the compounds of the formula (I) 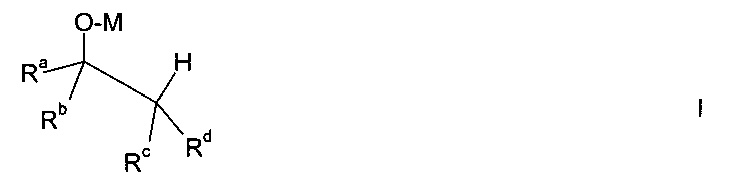 where
M denotes an alkali metal, an alkaline-earth metal halide or an H atom, and
R^{b}, R^{c}, R^{d}, independently of one another, denote an optionally substituted aliphatic or aromatic radical, which may have one or more heteroatoms, where one, two or three radicals from the group R^{a} to R^{d} may also denote H, and where two or more of the radicals R^{a}, R^{b}, R^{c} and/or R^{d} may be connected to one another,
R^{a} has a meaning of the formula la 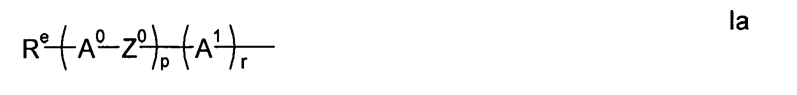 in which
R^{e} denotes an alkyl radical having 1 to 15 C atoms which is unsubstituted, monosubstituted or polysubstituted by CN and/or halogen, where, in addition, one or more CH₂ groups in these radicals may be replaced by -O-, -S-, -CH=CH-, -C≡C-, -OC-O- and/or -O-CO- and/or, in addition, one or more CH groups may be replaced by N or P in such a way
that two O atoms are not linked directly to one another, or, if r and/or p are not 0, also H, halogen, CN, SF₅ or NCS,
A⁰, A¹ each, independently of one another, denote
a) a 1,4-cyclohexenylene or 1,4-cyclohexylene radical, in which one or two non-adjacent CH₂ groups may be replaced by -O- or -S-,
b) a 1,4-phenylene radical, in which one or two CH groups may be replaced by N,
c) a radical from the group piperidine-1,4-diyl, 1,4-bicyclo-[2.2.2]octylene, phenanthrene-2,7-diyl, naphthalene-2,6-diyl, decahydronaphthalene-2,6-diyl and 1,2,3,4-tetrahydronaphthalene-2,6-diyl,
d) a divalent radical from the group furan, pyrrole, thiophene, pyrazole, imidazole, 1,2-oxazole, 1,3-oxazole, thiazole, pyridine, pyridazine, pyrimidine, pyrazine, 2H-pyran, 4H-pyran, purine, pteridine, 1H-azepine, 3H-1,4-diazepine, indole, benzofuran, benzothiophene, quinoline, isoquinoline, phenazine, phenoxazine, phenothiazine and 1,4-benzodiazepine,
where the radicals a), b), c) and d) may be mono- or polysubstituted by R^{e}, in particular by halogen and/or CN,
Z⁰ denotes -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- or a single bond,
p denotes 0, 1, 2 or 3 and
r denotes 1 or 2,
**characterised in that** the dehydration is carried out in ionic liquids of the general formula K⁺A⁻, where the anion A⁻ of the ionic liquid is selected from the group [HSO₄]⁻, [SO₄]²⁻ , [NO₃]⁻ [BF₄]⁻, [(R_{F})BF₃]⁻ [(R_{F})₂BF₂]⁻, [(RF)₃BF]⁻, [(R_{F})₄B]⁻, [B(CN)₄]⁻, [PO₄]³⁻, [HPO₄]²⁻, [H₂PO₄]⁻, [alkyl-OPO₃]²⁻, [(alkyl-O)₂PO₂]⁻, [alkyl-PO₃]⁻, [R_{F}PO₃]⁻, [(alkyl)₂PO₂]⁻, [(RF)₂PO₂]⁻, [R_{F}SO₃]⁻, [alkyl-SO₃]⁻, [aryl-SO₃]⁻, [alkyl-OSO₃]⁻, [R_{F}C(O)O]⁻, [(R_{F}SO₂)₂N]⁻, {[(R_{F})₂P(O)]₂N}⁻, Cl⁻ and/or Br⁻, where R_{F} has the meaning fluorinated alkyl
(CnF₂ₙ₋ₓ₊₁Hₓ)
where n = 1-12 and x = 0-7, where, for n = 1, x should be = 0 to 2, and/or fluorinated aryl or alkylaryl, and the cations are selected from the group of the ammonium, phosphonium, thiouronium, guanidinium cations or heterocyclic cations.

2. Process according to Claim 1, **characterised in that** the ammonium cations conform to the formula (1),
[NR₄]⁺ (1),
where
R in each case, independently of one another, denotes
H, where all substituents R cannot simultaneously be H, straight-chain or branched alkyl having 1-20 C atoms or
saturated or unsaturated C₃- to C₇-cycloalkyl, which may be substituted by C₁- to C₆-alkyl groups,
or **in that** the phosphonium cations conform to the formula (2),
[PR²₄]⁺ (2),
where
R² in each case, independently of one another, denotes H,
straight-chain or branched alkyl having 1-20 C atoms or
saturated or unsaturated C₃- to C₇-cycloalkyl, which may be substituted by C₁- to C₆-alkyl groups,
or **in that** the thiouronium cations conform to the formula (3)
[(R³R⁴N)-C(=SR⁵)(NR⁶R⁷)]⁺ (3),
where
R³ to R⁷ each, independently of one another, denote hydrogen, where hydrogen is excluded for R⁵,
straight-chain or branched alkyl having 1 to 20 C atoms or
saturated or unsaturated C₃- to C₇-cycloalkyl, which may be substituted by C₁- to C₆-alkyl groups,
or **in that** the guanidinium cations conform to the formula (4)
[C(NR⁸R⁹)(NR¹⁰R¹¹)(NR¹²R¹³)]⁺ (4),
where
R⁸ to R¹³ each, independently of one another, denote hydrogen,
straight-chain or branched alkyl having 1 to 20 C atoms or
saturated or unsaturated C₃- to C₇-cycloalkyl, which may be substituted by C₁- to C₆-alkyl groups, or
**in that** the heterocyclic cations conform to the formula (5)
[HetN]⁺ (5),
where
HetN⁺ denotes a heterocyclic cation selected from the group 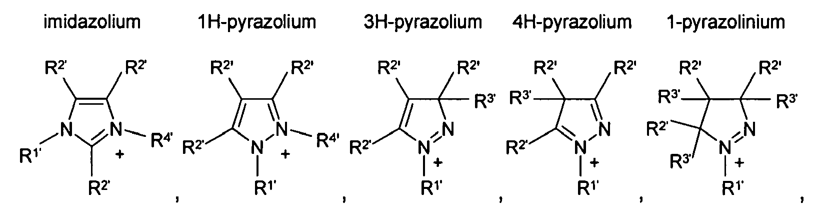 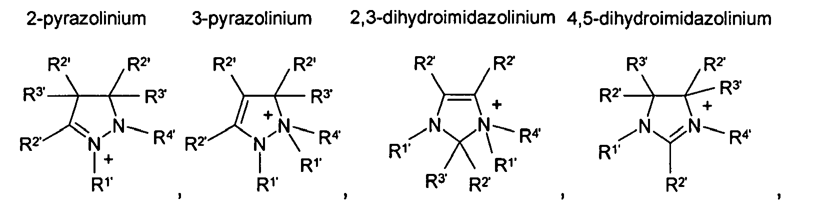 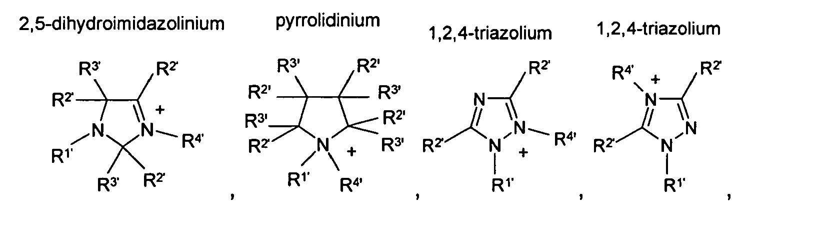 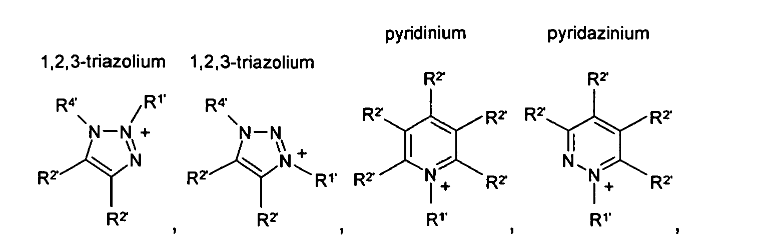 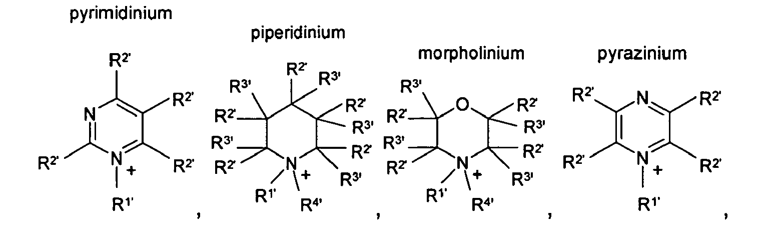 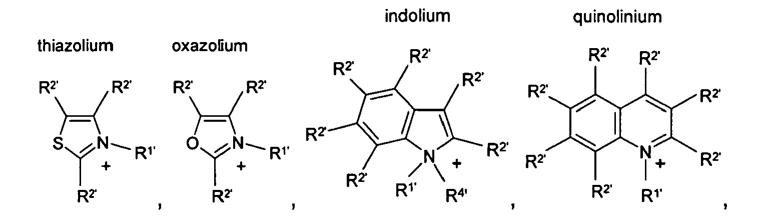 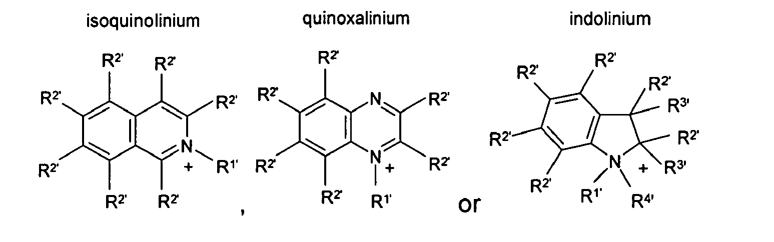
where the substituents
R^{1'} to R^{4'} each, independently of one another, denote hydrogen,
straight-chain or branched alkyl having 1-20 C atoms or
saturated or unsaturated C₃- to C₇-cycloalkyl, which may be substituted by C₁- to C₆-alkyl groups.

3. Process according to Claim 1 or 2, **characterised in that** the process temperature is 0 to 170°C.

4. Process according to Claim 1, **characterised in that** M denotes H, Li, MgCl, MgBr or Mgl.

5. Process according to one of the preceding claims, **characterised in that** one, two or three radicals from the group R^{b}, R^{c}, R^{d} have, independently of one another, a meaning of the formula la, and any other radicals from the group R^{b}, R^{c}, R^{d} denote H.

## Revendications

1. Procédé de déshydratation de polyalcools, d'alcools ou d'alcoolates ayant au moins un groupement CH en position α par rapport à la fonction alcoolate ou alcool pour donner des alcènes, où l'alcool est choisi parmi les composés de formule (I) 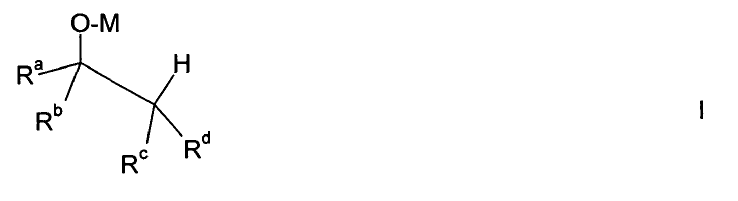 dans laquelle
M désigne un halogénure de métal alcalin, de métal alcalino-terreux ou un atome de H, et
R^{b}, R^{c}, R^{d}, désignent, indépendamment les uns des autres, un radical aliphatique ou aromatique éventuellement substitué, qui peut posséder un ou plusieurs hétéroatomes, où un, deux ou trois radicaux issus du groupe R^{a} à R^{d} peuvent aussi désigner H, et où deux, ou plus, parmi les radicaux R^{a}, R^{b}, R^{c} et/ou R^{d} peuvent être reliés l'un à l'autre,
R^{a} revêt une signification de formule la 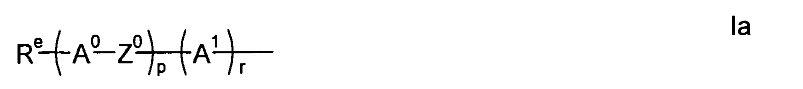 dans laquelle
R^{e} désigne un radical alkyle ayant 1 à 15 atomes de C qui est non substitué, monosubstitué ou polysubstitué par CN et/ou halogène, où, de plus, un ou plusieurs groupements CH₂ dans ces radicaux peuvent être remplacés par -O-, -S-, -CH=CH-, -C≡C-, -OC-O- et/ou -O-CO- et/ou, de plus, un ou plusieurs groupements CH peuvent être remplacés par N ou P de sorte que deux atomes de O ne soient pas liés directement l'un à l'autre, ou, si r et/ou p ne valent pas 0, également H, halogène, CN, SF₅ ou NCS,
A⁰, A¹ désignent chacun, indépendamment l'un de l'autre,
a) un radical 1,4-cyclohexénylène ou 1,4-cyclohexylène, dans lequel un ou deux groupements CH₂ non adjacents peuvent être remplacés par -O- ou -S-,
b) un radical 1,4-phénylène, dans lequel un ou deux groupements CH peuvent être remplacés par N,
c) un radical issu du groupe pipéridine-1,4-diyle, 1,4-bicyclo[2.2.2]octylène, phénanthrène-2,7-diyle, naphtalène-2,6-diyle, décahydronaphtalène-2,6-diyle et 1,2,3,4-tétrahydronaphtalène-2,6-diyle,
d) un radical divalent issu du groupe furane, pyrrole, thiophène, pyrazole, imidazole, 1,2-oxazole, 1,3-oxazole, thiazole, pyridine, pyridazine, pyrimidine, pyrazine, 2H-pyranne, 4H-pyranne, purine, ptéridine, 1 H-azépine, 3H-1,4-diazépine, indole, benzofurane, benzothiophène, quinoléine, isoquinoléine, phénazine, phénoxazine, phénothiazine et 1,4-benzodiazépine,
où les radicaux a), b), c) et d) peuvent être mono- ou polysubstitués par R^{e}, en particulier par halogène et/ou CN,
Z⁰ désigne -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- ou une liaison simple,
p désigne 0, 1, 2 ou 3 et
r désigne 1 ou 2,
**caractérisé en ce que** la déshydratation est effectuée dans des liquides ioniques de formule générale K⁺A⁻, où l'anion A⁻ du liquide ionique est choisi parmi le groupe [HSO₄]⁻, [SO₄]²⁻, [NO₃]⁻, [BF₄]⁻, [(R_{F})BF₃]⁻, [(R_{F})₂BF₂]⁻. [(R_{F})₃BF]⁻, [(R_{F})₄B]⁻, [B(CN)₄]⁻, [PO₄]³⁻, [HPO₄]²⁻, [H₂PO₄]⁻, [alkyl-OPO₃]²⁻, [(alkyl-O)₂PO₂]⁻, [alkyl-PO₃]⁻, [R_{F}PO₃]⁻, [(alkyl)₂PO₂]⁻, [(R_{F})₂PO₂]⁻, [R_{F}SO₃]⁻, [alkyl-SO₃]⁻, [aryl-SO₃]⁻, [alkyl-OSO₃]⁻, [R_{F}C(O)O]⁻, [(R_{F}SO₂)₂N]⁻, {[(R_{F})₂P(O)]₂N}⁻, Cl⁻ et/ou Br⁻, où R_{F} revêt la signification alkyle fluoré
(CₙF₂ₙ₋ₓ₊₁Hₓ)
où n = 1-12 et x = 0-7, où, pour n = 1, x doit être = 0 à 2,
et/ou aryle ou alkylaryle fluoré, et les cations sont choisis dans le groupe constitué par les cations ammonium, phosphonium, thiouronium, guanidinium ou les cations hétérocycliques.

2. Procédé selon la revendication 1, **caractérisé en ce que** les cations ammonium répondent à la formule (1),
[NR₄]⁺ (1),
dans laquelle
R désigne dans chaque cas, indépendamment l'un de l'autre,
H, où tous les substituants R ne peuvent être simultanément H, alkyle à chaîne linéaire ou ramifiée ayant 1-20 atomes de C ou C₃- à C₇-cycloalkyle saturé ou insaturé, pouvant être substitué par des groupements C₁- à C₆-alkyle,
ou **en ce que** les cations phosphonium répondent à la formule (2),
[PR²₄]⁺ (2),
dans laquelle
R² désigne dans chaque cas, indépendamment l'un de l'autre, H,
alkyle à chaîne linéaire ou ramifiée ayant 1-20 atomes de C ou C₃- à C₇-cycloalkyle saturé ou insaturé, pouvant être substitué par des groupements C₁- à C₆-alkyle,
ou **en ce que** les cations thiouronium répondent à la formule (3)
[(R³R⁴N)-C(=SR⁵)(NR⁶R⁷)]⁺ (3),
dans laquelle
R³ à R⁷ désignent chacun, indépendamment l'un de l'autre, hydrogène, où hydrogène est exclu pour R⁵,
alkyle à chaîne linéaire ou ramifiée ayant 1 à 20 atomes de C ou
C₃- à C₇-cycloalkyle saturé ou insaturé, pouvant être substitué par des groupements C₁- à C₆-alkyle,
ou **en ce que** les cations guanidinium répondent à la formule (4)
[C(NR⁸R⁹)(NR¹⁰R¹¹)(NR¹²R¹³)]⁺ (4),
dans laquelle
R⁸ à R¹³ désignent chacun, indépendamment l'un de l'autre, hydrogène,
alkyle à chaîne linéaire ou ramifiée ayant 1 à 20 atomes de C ou
C₃- à C₇-cycloalkyle saturé ou insaturé, pouvant être substitué par des groupements C₁- à C₆-alkyle, ou
ou **en ce que** les cations hétérocycliques répondent à la formule (5)
[HétN]⁺ (5),
dans laquelle
HétN⁺ désigne un cation hétérocyclique choisi dans le groupe constitué par 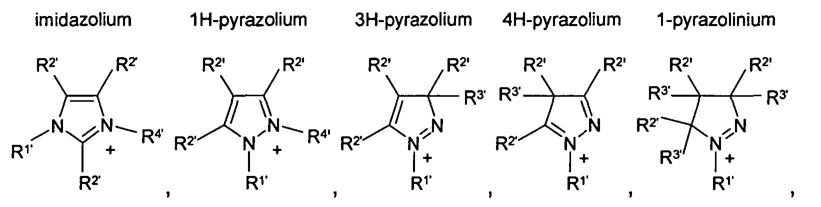 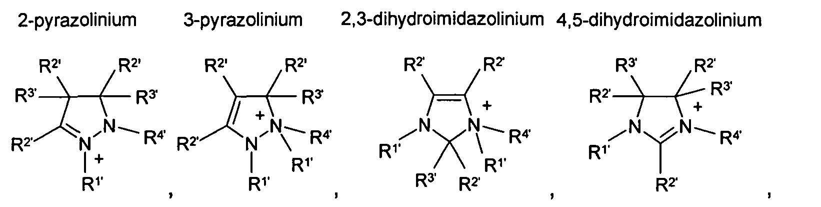 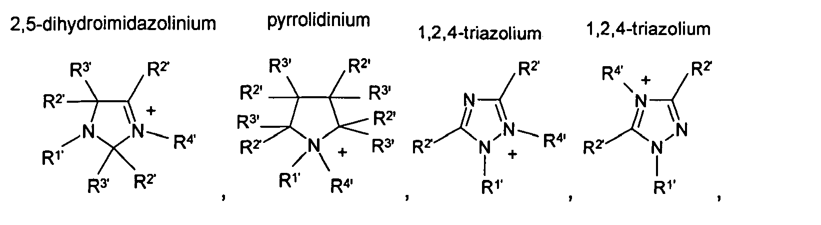 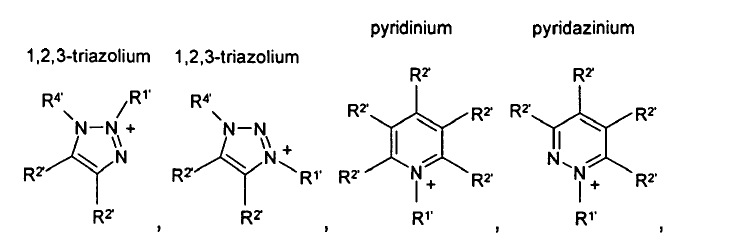 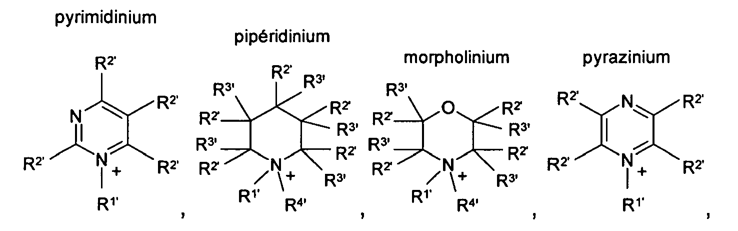 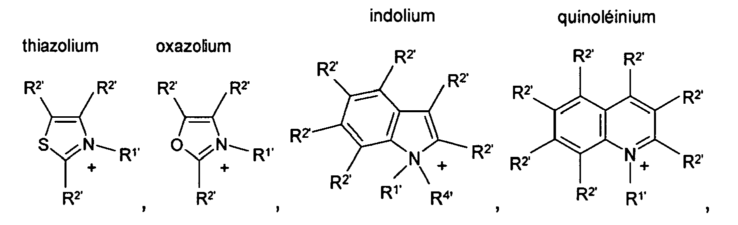 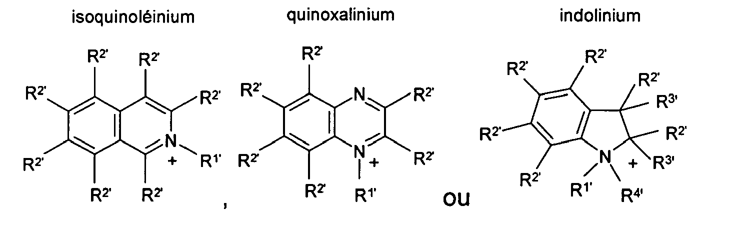 où les substituants
R^{1'} à R^{4'} désignent chacun, indépendamment l'un de l'autre, hydrogène,
alkyle à chaîne linéaire ou ramifiée ayant 1-20 atomes de C ou
C₃- à C₇-cycloalkyle saturé ou insaturé, pouvant être substitué par des groupements C₁- à C₆-alkyle.

3. Procédé selon la revendication 1 ou 2, **caractérisé en ce que** la température du procédé va de 0 à 170°C.

4. Procédé selon la revendication 1, **caractérisé en ce que** M désigne H, Li, MgCl, MgBr ou Mgl.

5. Procédé selon l'une quelconque des revendications précédentes, **caractérisé en ce que** un, deux ou trois radicaux issus du groupe R^{b}, R^{c}, R^{d} revêtent, indépendamment les uns des autres, une signification de formule la, et tout autre radical issu du groupe R^{b}, R^{c}, R^{d} désigne H.
